# EUROPEAN PATENT APPLICATION

(11) **EP 3 011 906 A1**
(43) Date of publication of application: **27.04.2016**
(21) Application number: 14813102.2
(22) Date of filing: 31.03.2014
(51) Int. Cl.: A61B 6/03, G01T 1/20, G01T 7/00

(54) **RADIATION DETECTION MODULE AND RADIATION DETECTION UNIT**

(30) Priority: 18.06.2013 JP 2013127544
(71) Applicant: Hamamatsu Photonics K.K., Hamamatsu-shi, Shizuoka 435-8558 (JP)
(72) Inventor: SUGIYA, Mitsutoshi, Hamamatsu-shi Shizuoka 435-8558 (JP); NAGURA, Keisuke, Hamamatsu-shi Shizuoka 435-8558 (JP)
(74) Representative: Nederlandsch Octrooibureau
(86) International application number: PCT/JP2014/059440
(87) International publication number: WO 2014/203589

(57) **Abstract**

A radiation detection module for a CT device that detects radiation includes a detecting section configured to detect radiation, a processing section configured to process a signal from the detecting section, and a heat radiating member thermally coupled to the processing section and configured to dissipate heat generated by the processing section. The heat radiating member has a plurality of fins. The plurality of fins is spaced apart from each other in a first direction along a slice direction of the CT device. Each of the plurality of fins has a plate shape extending so as to intersect the first direction.

## Description

### Technical Field

The present invention relates to a radiation detection module and a radiation detection unit.

### Background Art

Conventionally, a radiation detection module for detecting radiation including X-ray, and a radiation detection unit equipped with the plurality of radiation detection modules are known (refer to Patent Literature 1, for example). Patent Literature 1 describes a detector module for detecting X-ray. The detector module is equipped with a plurality of semiconductor detectors and a metal block on which the plurality of semiconductor detectors is attached. Each of the semiconductor detectors has a scintillation crystal and a photodiode.

Patent Literature 1 describes a detector assembly equipped with the above-described plurality of detector modules. The detector assembly includes the plurality of above-described detector modules and an arcuate supporting reference spine provided in a channel direction. The plurality of detector modules is mounted on the supporting reference spine in the channel direction.

### Citation List

### Patent Literature

Patent Literature 1: Japanese Unexamined Patent Application Publication (Translation of PCT Application) No. 09-508305

### Summary of Invention

### Technical Problem

In use of the above-described radiation detection module and the radiation detection unit, heat is generated in such as a processing section that processes a signal from a detecting section detecting radiation. Temperature variation in the radiation detection module due to heat generation may lower radiation detection accuracy. Accordingly, it is desirable to improve heat radiation performance in the radiation detection module and the radiation detection unit.

The present invention is directed to solve the problem as described above. An object of the invention is to provide a radiation detection module and a radiation detection unit that are capable of improving heat radiation performance.

### Solution to Problem

A radiation detection module according to an aspect of the present invention is a radiation detection module for a CT device that detects radiation. The radiation detection module includes a detecting section configured to detect radiation, a processing section configured to process a signal from the detecting section, and a heat radiating member thermally coupled to the processing section and is configured to dissipate the heat generated by the processing section. The heat radiating member includes a plurality of fins. The plurality of fins is spaced apart from each other in a first direction along a slice direction of the CT device. Each of the plurality of fins has a plate shape extending so as to intersect the first direction.

In the radiation detection module, the plurality of fins provided at the heat radiating member is spaced apart from each other in the first direction along the slice direction of the CT device (namely, direction orthogonal to a gantry rotating direction of the CT device). This generates an air flow path between the fins adjacent to each other when the gantry is rotated. Accordingly, this improves heat radiation performance. Each of the plurality of fins has a plate shape extending so as to intersect the first direction along the slice direction (namely, plate shape extending so as not to be orthogonal to the gantry rotating direction). This generates an air flow path and reduces air resistance at a time of gantry rotation, compared with a case where each of the plurality of fins has a shape extending so as to be orthogonal to the gantry rotation direction. Accordingly, this decreases a power required to rotate the gantry.

Between the processing section and the heat radiating member, high thermal conductive resin that thermally couples the processing section and the heat radiating member may be arranged. In this case, the heat generated in the processing section is transmitted appropriately to the heat radiating member via the high thermal conductive resin. Accordingly, this further improves heat radiation performance.

The radiation detection module may further include a base plate configured to support the detecting section and the processing section. The heat radiating member may include a coupling portion thermally coupled to the processing section via the high thermal conductive resin, and a fixed portion fixed to the base plate. The fixed portion may protrude toward the base plate side with respect to the coupling portion. The processing section and the high thermal conductive resin may be arranged at a gap formed between the base plate and the coupling portion by the fixed portion. In this case, it is possible to implement thermal coupling and mechanical fixation using a simple configuration.

The detecting section may include a scintillator configured to generate scintillation light in response to the incident radiation, and a detecting element configured to detect scintillation light from the scintillator. The amount of light emission of the scintillator varies depending on the temperature of the scintillator. The above-described configuration makes it possible to suppress transmission of the heat generated in the processing section to the scintillator, and suppress temperature variation of the scintillator.

The radiation detection unit according to an aspect of the present invention is the radiation detection unit for a CT device, equipped with the plurality of above-described radiation detection modules. The radiation detection unit includes a frame extending in the first direction and provided with the plurality of heat radiating members along the first direction. Each of the heat radiating members is mounted on the frame via a rod member so as to be spaced apart from the frame.

The radiation detection unit, as described above, makes it possible to improve heat radiation performance and decrease the power required to rotate the gantry. In the radiation detection unit, the heat radiating member of each of the radiation detection modules is mounted on the frame via a supporting member so as to be spaced apart from the frame. This enables aligning the incident surfaces among the plurality of radiation detection modules, and simultaneously absorbing a dimensional error and an assembly error of each of the components, at a gap between the heat radiating member and the frame. Accordingly, this enables aligning the incident surfaces among the plurality of radiation detection modules and improving positional accuracy.

### Advantageous Effects of Invention

According to the present invention, it is possible to provide a radiation detection module and a radiation detection unit that are capable of improving heat radiation performance.

### Brief Description of Drawings

FIG. 1 is a schematic diagram illustrating a CT device equipped with a plurality of radiation detection units according to an embodiment.
FIG. 2 is a perspective view of the radiation detection unit illustrated in FIG. 1.
FIG. 3 is a side view of the radiation detection module illustrated in FIG. 2.
FIG. 4 is a diagram viewed in the arrow direction of the IV-IV line in FIG. 3.
FIG. 5 is a perspective view of a heat radiating member illustrated in FIG. 4.

### Description of Embodiments

Hereinafter, a radiation detection module and a radiation detection unit according to an embodiment will be described with reference to the drawings. The same reference signs are given to same or similar components, and duplicate descriptions will be omitted.

### [CT device]

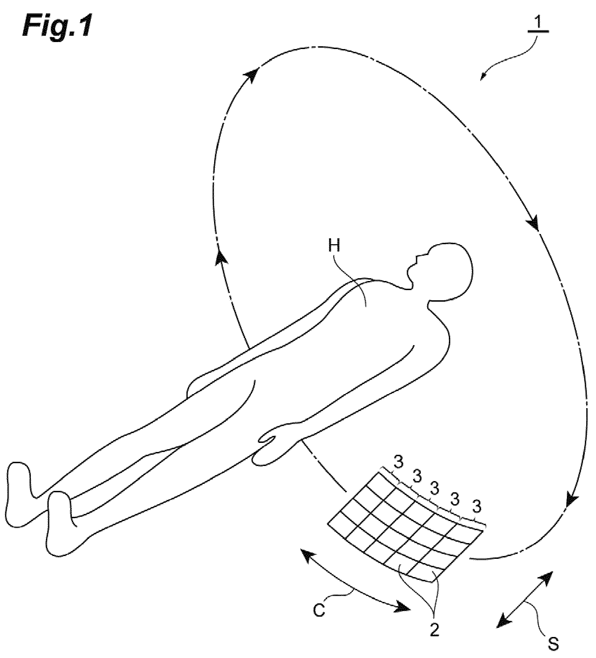

FIG. 1 is a schematic diagram illustrating a CT device equipped with the plurality of radiation detection units according to an embodiment. As illustrated in FIG. 1, a CT (Computed Tomography) device 1 irradiates radiation (X-ray, γ-ray, or the like) from a radiation source (not illustrated) to a subject H, and detects the radiation that has been transmitted through the subject H is detected by a plurality of detection modules (radiation detection module) 2. The plurality of detection modules 2 are fixed to a rotation mechanism (gantry) (not illustrated). The plurality of detection modules 2 rotates in a gantry rotating direction (channel direction) C and linearly moves in a slice direction (body axial direction) S.

The plurality of detection modules 2 is arranged in each of the channel direction C and the slice direction S. A detection unit (radiation detection unit) 3 is equipped with the plurality of detection modules 2 that is arranged in the slice direction S.

### [Radiation Detection Unit]

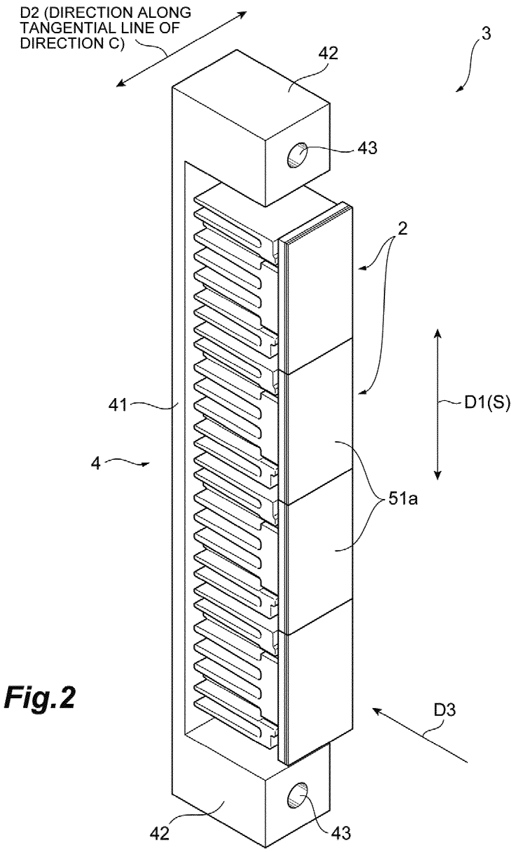

FIG. 2 is a perspective view of the radiation detection unit illustrated in FIG. 1. Herein, the direction along the slice direction S is a first direction D 1; the direction along a tangential line of the channel direction C (direction orthogonal to the first direction D1) is a second direction D2; and the direction along a normal of an incident surface 51a (described below) of the detection module 2 is a third direction D3.

As illustrated in FIG. 2, the detection unit 3 includes the plurality of above-described detection modules 2 and a frame 4. The frame 4 extends in the first direction D1. Specifically, the frame 4 includes a supporting portion 41 extending in the first direction D1, and in-frame abutment portions 42, 42 each of which is located on each of the end portions of the supporting portion 41 in the first direction D1.

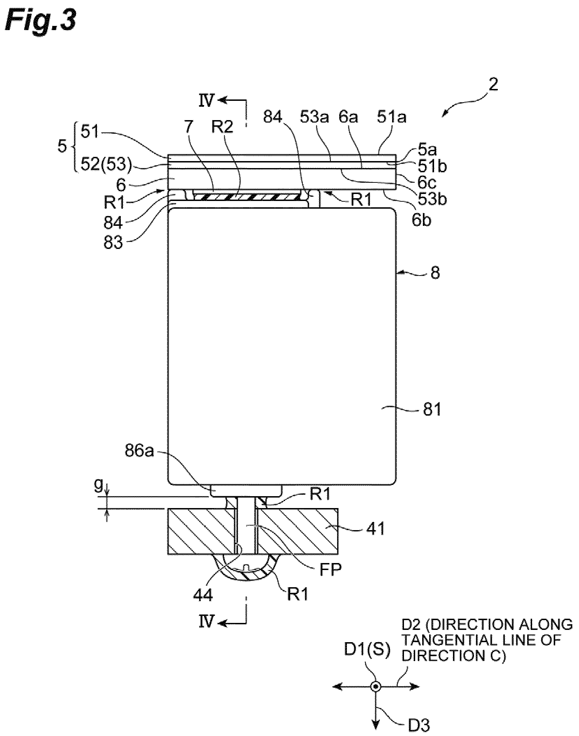

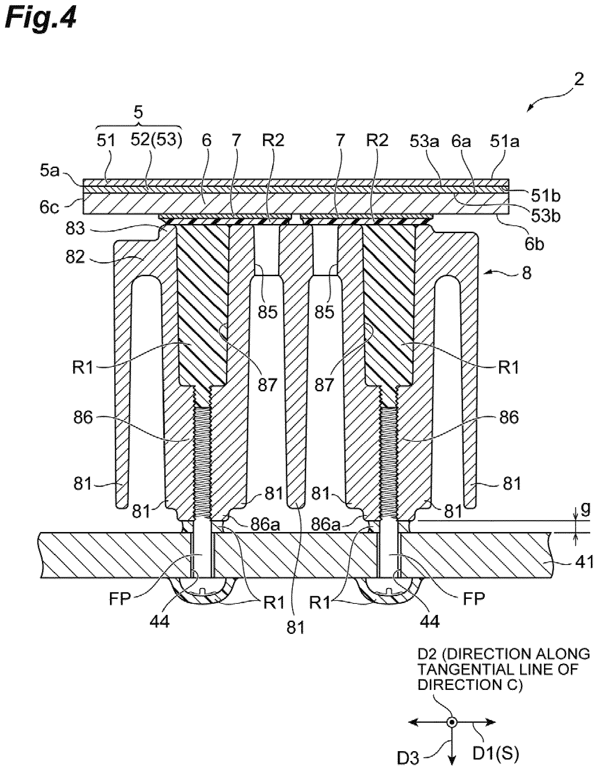

The supporting portion 41 has a long plate-like shape. The in-frame abutment portion 42 has a rectangular parallelepiped shape and protrudes from one surface of the supporting portion 41. The in-frame abutment portion 42 has a through hole 43. The plurality of detection modules 2 is mounted on the supporting portion 41 in the first direction D1. The detection module 2 is mounted spaced apart from one surface of the supporting portion 41 (refer to FIGS. 3 and 4; details will be described below).

### [Radiation Detection Module]

FIG. 3 is a side view of the radiation detection module illustrated in FIG. 2. FIG. 4 is diagram viewed in the arrow direction of the IV-IV line in FIG. 3. As illustrated in FIGS. 3 and 4, the detection module 2 includes a detecting section 5, a supporting base plate 6, a processing section 7, and a heat radiating member 8.

The detecting section 5 includes a scintillator 51 and a photodiode array (detecting element) 52. The scintillator 51 has a rectangular (specifically, equiangular quadrilateral) plate shape (refer to FIG. 2). The scintillator 51 extends in the first direction D1 and the second direction D2. The scintillator 51 includes an incident surface 51a on which radiation is incident, and an emission surface 51b that is located on the opposite side of the incident surface 51 a and emits scintillation light in response to incident radiation. Each of the incident surface 51a and the emission surface 51b extends in the first direction D1 and the second direction D2. The scintillator 51 is, for example, a CsI doped with Tl, or the like. The CsI has a structure including a forest of large number of needle-shaped crystals (columnar crystals).

The photodiode array 52 detects scintillation light from the scintillator 51. The photodiode array 52 includes a plurality of photodiodes and a semiconductor substrate 53 that includes the plurality of photodiodes. The semiconductor substrate 53, when viewed in the third direction D3, has substantially the same shape as the scintillator 51, or the rectangular plate shape that is slightly larger than the scintillator 51. The semiconductor substrate 53 extends in the first direction D1 and the second direction D2. The plurality of photodiodes is arranged two-dimensionally on the semiconductor substrate 53. The semiconductor substrate 53 includes a first surface 53a on which scintillation light from the scintillator 51 is incident and a second surface 53b located on the opposite side of the first surface 53a. Each of the first surface 53a and the second surface 53b extends in the first direction D1 and the second direction D2. The scintillator 51 is located on the first surface 53a.

The semiconductor substrate 53 is formed of silicon or the like. The photodiode array 52 is, for example, a back-illuminated type in which, for example, a photosensitive region is located on the second surface 53b side. Note that the photodiode array 52 may be a front-illuminated type in which the photosensitive region thereof is located on the first surface 53a. When the photodiode array 52 is a front-illumination type, the photodiode array 52 and a land electrode (described below) of the supporting base plate 6 may be coupled via a through electrode formed inside the semiconductor substrate 53, or may be coupled by the wire bonding.

The photodiode array 52 is coupled to the emission surface 51b of the scintillator 51 via an optically transparent optical coupling agent with respect to the scintillation light from the scintillator 51. The photodiode array 52 has sensitivity, for example, ranging from an ultraviolet region to a near-infrared region. On the detecting section 5 as described above, a portion that is exposed in the first direction D1 and the second direction D2 that are orthogonal to the third direction (portion along the third direction D3) is a side portion 5a.

The supporting base plate 6 supports the detecting section 5 and the processing section 7. The supporting base plate 6 has substantially the same shape of a rectangular plate shape as the semiconductor substrate 53, when viewed in the third direction D3. The supporting base plate 6 extends in the first direction D1 and the second direction D2. The supporting base plate 6 has a first surface 6a that supports the detecting section 5, and a second surface 6b that is located on the opposite side of the first surface 6a and supports the processing section 7. Each of the first surface 6a and the second surface 6b extends in the first direction D 1 and the second direction D2. On the supporting base plate 6, a portion exposed in the first direction D 1 and the second direction D2 (portion along the third direction D3) is a side portion 6c. The supporting base plate 6 and the semiconductor substrate 53 are integrated.

On each of the first surface 6a and the second surface 6b of the supporting base plate 6, a land electrode is formed. On the land electrode of the first surface 6a, a photodiode of the semiconductor substrate 53 is coupled via a bump electrode. On the land electrode of the second surface 6b, the processing section 7 is coupled via a bump electrode. Inside the supporting base plate 6, a conductor pattern is formed for coupling the land electrodes of the first surface 6a and the second surface 6b to each other.

The supporting base plate 6, for example, is formed by laminating a plurality of green sheets containing ceramic and by firing the laminate structure. The supporting base plate 6 may be formed with an organic material (glass epoxy resin, or the like).

The processing section 7 processes a signal from the photodiode array 52. As illustrated in FIG. 4, the plurality of processing sections 7 is provided. The processing section 7, when viewed in the third direction D3, has a rectangular (specifically, equiangular quadrilateral) plate shape that is smaller than the supporting base plate 6. The plurality of processing sections 7, 7 is spaced apart from each other in the first direction D1. The processing section 7 is, for example, an application specific integrated circuit (ASIC).

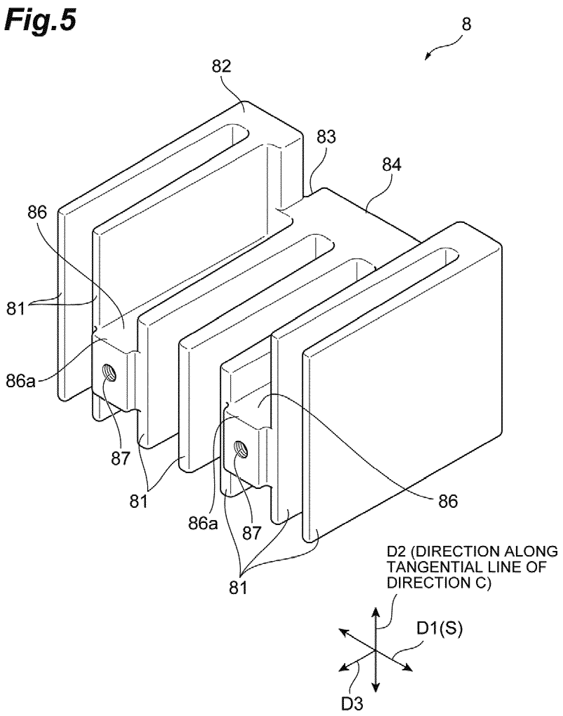

The heat radiating member 8 is thermally coupled to the processing sections 7, 7 and dissipates the heat generated by the processing sections 7, 7. FIG. 5 is a perspective view of the heat radiating member in FIG. 4. As illustrated in FIGS. 4 and 5, the heat radiating member 8 includes a plurality of fins 81. Specifically, the cross section of the heat radiating member 8 in the first and third directions D1 and D3 has a substantially comb-shape (see FIG. 4). A portion coupling a plurality of comb teeth is a base 82. Portions of the comb teeth are the above-described fins 81. The heat radiating member 8, when viewed in the third direction, is smaller than the supporting base plate 6 and is substantially U-shaped (refer to FIG. 5). Examples of a material for forming the heat radiating member 8 include Al, Cu, or brass.

The base 82 has a substantially plate shape. The base 82 extends in the first direction D1 and the second direction D2. The processing sections 7, 7 are thermally coupled at the base 82. Specifically, at the base 82, the center portion in the first direction D1 protrudes toward the processing section 7 side with respect to the both end portions. The protruding portion is a coupling portion 83 to which the processing sections 7, 7 are to be coupled. As illustrated in FIG. 3, a pair of fixed portions 84, 84 protrudes toward the supporting base plate 6 side from the coupling portion 83.

The fixed portion 84 has a substantially rectangular parallelepiped shape and extends in the first direction D 1. The pair of fixed portions 84, 84 is spaced apart from each other in the second direction D2 and is located at both end portions of the coupling portion 83 in the second direction D2. The protrusion height of the fixed portion 84 is larger than the thickness of the processing section 7. The fixed portion 84 is fixed to the second surface 6b of the supporting base plate 6 by resin (first resin) R1. The resin R1, for example, may be an epoxy resin-based adhesive. The above-mentioned processing sections 7, 7 are arranged at a gap between the supporting base plate 6 and the coupling portion 83 formed by the fixed portions 84, 84.

As illustrated in FIG. 4, a plurality of through holes 85, 85 is provided at the coupling portion 83 corresponding to the number of processing sections 7. The through holes 85, 85 are spaced apart from each other in the first direction D1. The through hole 85 is provided between the pair of opposing fins 81, 81 in the first direction D1. The through hole 85 and the processing section 7 overlap with each other when viewed in the third direction D3.

Between the coupling portion 83 and the processing section 7, resin (second resin) R2 is sandwiched. For the resin R2, highly thermal conductive resin (for example, silicone resin) can be used. For example, highly thermally conductive resin having a higher thermal conductivity than that of the resin R1 can be used as the resin R2. The resin R2, for example, can be arranged in the following manner. First, the supporting base plate 6 and the fixed portion 84 are bonded with the resin R1. Subsequently, the resin R2 is inserted through the above-mentioned through holes 85, 85 into a space between the processing section 7 and the coupling portion 83.

The fin 81 protrudes toward the opposite side of the processing section 7 from the base portion 82 in the third direction D3. The Fin 81 has a plate shape extending so as to intersect the first direction D1. More specifically, the fin 81 has a plate shape extending so as to be substantially orthogonal to the first direction D1. In other words, the fin 81 has a plate shape extending in the second and third directions D2 and D3. In some gaps among the gaps between the pairs of opposing fins 81, 81, a coupling portion 86 that couples the pair of opposing fins 81, 81 is provided (see FIG. 5).

A plurality of (specifically, two) coupling portions 86 is provided in the first direction D1. The coupling portion 86 is provided at a gap that overlaps with the processing section 7 when viewed in the third direction D3. The coupling portions 86, 86 are arranged symmetrically with respect to a center of the detection module 2 in the first direction D1. The coupling portions 86, 86 are provided on the outer side, in the first direction D1, than the through holes 85, 85 into which the resin R2 is inserted as described above.

An end portion of the coupling portion 86 (lower end portion in FIGS. 3 and 4) protrudes more than an end portion of the fin 81. The end portion of the coupling portion 86 is located on the opposite side of the incident surface 51 a on the detection module 2, and functions as a mounting portion 86a.

The coupling portion 86 has a through hole 87 in the third direction D3. The through hole 87 has a larger diameter at a portion on the base 82 side than the portion on the mounting portion 86a side. The through hole 87 has an internal thread that is formed at a portion on the mounting portion 86a side. On the detection module 2, a flexible flat cable (FFC) 9 is attached for outputting signals to the outside (not illustrated).

The above-described detection module 2 and the frame 4 are attached to each other via a rod-shaped supporting pin (rod member) FP. Specifically, a through hole 44 is provided on the supporting portion 41 of the frame 4 at a position corresponding to the through hole 87 of the heat radiating member 8. An external thread is formed on the supporting pin FP. The supporting pin FP is inserted through the through hole 44 of the supporting portion 41 and is screwed into the through hole 87 of the heat radiating member 8. A gap g exists between the supporting portion 41 and the coupling portion 86.

For fixing the detection module 2 and the frame 4, adhesive is used. As the adhesive, the above-described resin R1 may be used, for example. Specifically, the resin R1 is inserted in the through hole 87. A gap may be formed between the resin R1 and the resin R2. Between the end portion of the coupling portion 86 and the supporting portion 41, the resin R1 is attached so as to cover the supporting pin FP. In the supporting pin FP, a portion (head) protruding from the supporting portion 41 is covered with the resin R1.

As described above, in the radiation detection module 2 according to the present embodiment, the plurality of fins 81 provided at the heat radiating member 8 is spaced apart from each other in the first direction D1 along the slice direction S (direction orthogonal to the gantry rotating direction C of the CT device) of the CT device 1. This generates an air flow path between the fins 81, 81 adjacent to each other, when the gantry is rotated. Accordingly, this improves heat radiation performance.

In the detection module 2, each of the plurality of fins 81 has a plate shape extending so as to intersect the first direction D1 along the slice direction S (namely, plate shape extending so as not to be orthogonal to the gantry rotating direction C). This generates an air flow path and reduces air resistance at the time of gantry rotation compared with a case where each of the plurality of fins 81 has a plate shape extending so as to be orthogonal to the gantry rotation direction C. Accordingly, this decreases a power required to rotate the gantry.

In the detection module 2, the resin R2 that is the high thermal conductive resin that thermally couples the processing section 7 and the heat radiating member 8 is arranged between the processing section 7 and the heat radiating member 8. Therefore, the heat generated in the processing section 7 is transmitted appropriately to the heat radiating member 8 via the resin R2. Accordingly, this further improves heat radiation performance.

The detection module 2 further includes the supporting base plate 6 configured to support the detecting section 5 and the processing section 7. The heat radiating member 8 includes the coupling portion 83 thermally coupled to the processing section 7 via the resin R2, and the fixed portion 84 fixed to the supporting base plate 6. The fixed portion 84 protrudes toward the supporting base plate 6 side with respect to the coupling portion 83. At the gap formed between the supporting base plate 6 and the coupling portion 83 by the fixed portion 84, the processing section 7 and the resin R2 are arranged. This makes it possible to implement thermal coupling and mechanical fixation using a simple configuration.

The detecting section 5 includes the scintillator 51 configured to generate scintillation light in response to the incident radiation, and the photodiode configured to detect scintillation light from the scintillator 51. The amount of light emission of the scintillator 51 varies depending on the temperature of the scintillator 51. The above-described configuration makes it possible to suppress transmission of the heat generated in the processing section 7 to the scintillator 51, and to suppress temperature variation in the scintillator 51.

The detection unit 3 according to the present embodiment is the detection unit 3 for a CT device, equipped with the plurality of above-described radiation detection modules 2. The detection unit 3 includes the frame 4 extending in the first direction D1 and provided with the plurality of heat radiating members 8 along the first direction D 1. Each of the heat radiating members 8 is mounted on the frame 4 via the supporting pin FP so as to be spaced apart from the frame.

The detection unit 3, as described above, makes it possible to improve heat radiation performance and decrease the power required to rotate the gantry. In the detection unit 3, the heat radiating member 8 of each of the detection modules 2 is mounted on the frame 4 via the supporting pin FP so as to be spaced apart from the frame 4. This enables aligning the incident surfaces 51 a among the plurality of detection modules 2, and simultaneously absorbing the dimensional error and the assembly error of each of the components, at the gap g between the heat radiating member 8 and the frame 4. Accordingly, this enables aligning the incident surfaces 51 a among the plurality of detection modules 2 and improving positional accuracy.

In the detection module 2, the pair of through holes 87, 87 is arranged symmetrically with respect to the center of the detection module 2 in the first direction D 1. The detection module 2 is fixed to the frame 4 via the pair of supporting pin FP, FP inserted into the pair of through holes 87, 87. This makes it possible to suppress a positional shift due to centrifugal force generated in gantry rotation.

The radiation detection module and the radiation detection unit according to the embodiment have been described as above. The present invention, however, is not limited to the above-described embodiment. According to the above-described embodiment, the detecting section 5 includes, for example, the scintillator 51 and the photodiode array 52. Alternatively, the detecting section 5 may be a direct detection type detecting element (element that uses a crystal such as CdTe, CdZnTe) that directly detects radiation. Configuration, the numbers, and the material of each of the components are not limited to the configuration, the numbers, and the material in the above-described embodiment but may be modified appropriately.

### Industrial Applicability

The present invention is applicable to a radiation detection module for a CT device.

### Reference Signs List

- 1: CT device

- 2: detection module (radiation detection module)
- 3: detection unit (radiation detection unit)
- 4: frame
- 5: detecting section
- 6: supporting base plate
- 7: processing section
- 8: heat radiating member
- 51: scintillator
- 52: photodiode array (detecting element)
- 81: fin
- FP: supporting pin (rod member)
- R1: resin (first resin)
- R2: resin (second resin)
- S: slice direction
- D1: first direction
- D2: second direction

## Claims

1. A radiation detection module for a CT device that detects radiation, the radiation detection module comprising:
a detecting section configured to detect radiation;
a processing section configured to process a signal from the detecting section; and
a heat radiating member thermally coupled to the processing section and configured to dissipate the heat generated by the processing section,
wherein the heat radiating member includes a plurality of fins,
the plurality of fins is spaced apart from each other in a first direction along a slice direction of the CT device, and
each of the plurality of fins has a plate shape extending so as to intersect the first direction.

2. The radiation detection module according to claim 1,
wherein high thermal conductive resin that thermally couples the processing section and the heat radiating member is arranged between the processing section and the heat radiating member.

3. The radiation detection module according to claim 2, further comprising a base plate configured to support the detecting section and the processing section,
wherein the heat radiating member includes
a coupling portion thermally coupled to the processing section via the high thermal conductive resin, and
a fixed portion fixed to the base plate,
the fixed portion protrudes toward the base plate side with respect to the coupling portion, and
the processing section and the high thermal conductive resin are arranged at a gap formed between the base plate and the coupling portion by the fixed portion.

4. The radiation detection module according to claim 1 or 2,
wherein the detecting section includes
a scintillator configured to generate scintillation light in response to the incident radiation, and
a detecting element configured to detect scintillation light from the scintillator.

5. A radiation detection unit for a CT device, equipped with a plurality of radiation detection modules according to any one of claims 1 to 4, the radiation detection unit comprising
a frame extending in the first direction and provided with the plurality of heat radiating members along the first direction,
wherein each of the heat radiating members is mounted on the frame via a rod member so as to be spaced apart from the frame.
